# EUROPEAN PATENT APPLICATION

(11) **EP 2 612 674 A1**
(43) Date of publication of application: **10.07.2013**
(21) Application number: 10856699.3
(22) Date of filing: 01.09.2010
(51) Int. Cl.: A61K 38/00, A61K 9/19, A61K 47/02, A61K 47/12, A61K 47/20

(54) **FREEZE-DRIED RECOMBINANT HUMAN BONE MORPHOGENETIC PROTEIN-2 PREPARATION**

(71) Applicant: Osteopharma Inc., Osaka 540-0026 (JP)
(72) Inventor: NAGAYA, Masahiro, Ikeda-shi Osaka 563-0031 (JP); SUGAMA, Kazushige, Takaishi-shi Osaka 592-0002 (JP); SEBALD, Walter, Wuerzburg 97074 (DE)
(74) Representative: von Kreisler Selting Werner
(86) International application number: PCT/JP2010/064967
(87) International publication number: WO 2012/029148

(57) **Abstract**

The present invention provides a freeze-dried preparation obtained by dissolving an active type human bone morphogenetic protein-2 homodimer added with an alanine residue to the N-terminus in a strong acid solution and freeze-drying the solution, a production method of the preparation, and a kit for regeneration and treatment of bone, which contains the preparation.

## Description

### Technical Field

The present invention relates to a freeze-dried human bone morphogenetic protein-2 variant preparation having improved preservation stability. More particularly, the present invention relates to a freeze-dried preparation of human bone morphogenetic protein-2 added with an alanine residue to the N-terminus.

### Background Art

Bone morphogenetic protein-2 (hereinafter to be indicated as "BMP-2") is a protein belonging to the transforming growth factor-β (TGF-β) superfamily, and plays important biological roles such as bone formation, organ formation (formation of tooth, heart, eye, cartilage and the like) and the like. BMP is known to include some members, and each member has highly-conserved cysteine residues that form 3 disulfide bonds (called "cysteine knot") and one intermolecular disulfide bond. BMP-2 is constituted with one cysteine knot, one α-helix and at least 4 β-sheets, per molecule, whereby the monomer can form a dimer. In other words, BMP-2 takes the form of a homodimer consisting of single BMP-2 as an active type.

BMP-2 is synthesized as a precursor BMP-2 protein of an inactive type homodimer that underwent sugar chain modification in a mammalian cell, extracellularly secreted and processed by a protease to be a mature protein of an active type homodimer. The active type homodimer BMP-2 protein binds to the receptor as a ligand of type I or type II receptor of BMP, whereby the signal of BMP-2 is transmitted intracellularly.

To produce BMP-2, a general gene recombination technique is used. A DNA encoding,BMP-2 is introduced into a prokaryotic or eucaryotic cell via a vector, and the DNA is expressed and translated in the cell to give recombinant BMP-2 (patent documents 1, 2 and 3, non-patent document 1). As mentioned above, BMP-2 needs to form a dimer structure to be an active type, during which a correct disulfide bond needs to be formed as well as the DNA needs to be folded in a correct steric configuration (patent document 4).

When recombinant BMP-2 is produced using a prokdaryotic cell, the produced recombinant protein does not have a sugar chain, unlike use of a eucaryotic cell. While many of the BMP members are known to retain biological activity even without a sugar chain, since BMP-2 is a protein having relatively high hydrophobicity, recombinant BMP-2 without a sugar chain is defective in that it easily forms coagulation and shows poor solubility in water from weak acidic to neutral range. As mentioned above, moreover, since BMP-2 has activity only when a properly-folded dimer is formed, when recombinant BMP-2 is produced using a prokaryotic cell, stabilization of BMP-2 during the production step of BMP-2 and the formulation step of a pharmaceutical product is the problem.

A gene recombinant protein expressed in *Escherichia coli* needs to have Met as an amino acid residue from which to read the N-terminus. However, Met may or may not be truncated. Therefore, by setting the N-terminal sequence of recombinant BMP-2 for Met-Ala (non-patent document 1), N-terminal Met is completely cleaved and gene recombinant BMP-2 with the N-terminus uniformly starting with Ala can be obtained. This Ala-BMP-2 forms a homodimer like BMP-2, and has a receptor binding activity, an *in vitro* ALP induction activity into the target cell, and an *in vivo* bone induction activity.
Such "active type human bone morphogenetic protein-2 homodimer added with an alanine residue to the N-terminus" has a uniform N-terminal structure, and therefore, when industrially produced as an active ingredient of a pharmaceutical product, it offers a merit for quality control.

In the meantime, non-patent document 1 describes production of the above-mentioned "active type human bone morphogenetic protein-2 homodimer added with an alanine residue to the N-terminus" by using a prokaryotic cell, and a freeze-dried product thereof in distilled water. However, the solubility of said freeze-dried product in water and saline was poor, and the product was not satisfactory as a pharmaceutical preparation.

### [Document List]

### [patent documents]

patent document 1: WO2002/013,840
patent document 2: WO2006/125,868
patent document 3: WO1999/18,196
patent document 4: EP433,225

### [non-patent document]

non-patent document 1: R•Ruppertetal., Eur J Biochem, 237:295-302(1996)

### SUMMARY OF THE INVENTION

### Problems to be Solved by the Invention

As stated in the above-mentioned "Background Art", recombinant human BMP-2 produced using a prokaryotic cell is defective in that it easily coagulates and has very low solubility in water or buffered aqueous solution having a neutral range pH, which constitutes one obstacle for practicalization of a BMP-2 preparation. On the other hand, such recombinant BMP-2 free of a sugar chain is advantageous in that it can be produced massively by using a prokaryotic cell that can be cultured easily and is superior in proliferative capacity. In addition, there is another advantage in that a negative influence of a hydrocarbonate different from the human type sugar chain on the human body does not need to be considered unlike recombinant human BMP-2 produced by a heterogeneous eucaryotic cell. However, there are many problems to be solved in recombinant human BMP-2 free of a sugar chain, in addition to the problems of coagulation and solubility, such as storage stability including decreased activity and precipitate formation during storage, and durable efficacy of BMP-2 such as *in vivo* bone regeneration and repair.

Accordingly, the present invention mainly aims to provide a BMP-2 preparation that has solved particularly the problems of coagulation, solubility and storage stability of recombinant BMP-2, from the above-mentioned problems. The present invention also aims to provide a production method of the BMP-2 preparation, and a kit for regeneration or treatment of bone, which contains said BMP-2 preparation. Means of Solving the Problems

The present inventors have conducted intensive studies in an attempt to solve the aforementioned problems and found that superior resolubility and preservation stability can be achieved by dissolving active type human bone morphogenetic protein-2 homodimer added with an alanine residue to the N-terminus (hereinafter to be indicated as "Ala-BMP-2") in a strong acid solution and formulating same as a freeze-dried preparation, which resulted in the completion of the present invention. Based on such finding, the present inventors have further studied and confirmed that the aforementioned freeze-dried preparation is superior in the solubility in pure water, which resulted in the completion of the present invention.
Accordingly, the present invention is as described below.
[1] A freeze-dried preparation obtained by freeze-drying a solution containing Ala-BMP-2 and a strong acid;
[2] the freeze-dried preparation of [1], wherein the strong acid is at least one kind selected from the group consisting of hydrochloric acid, citric acid, trifluoroacetic acid and mesyl acid;
[3] the freeze-dried preparation of [1] or [2], wherein the strong acid has pH 0.2 - 5.1 at 25°C;
[4] a method of producing a freeze-dried preparation containing Ala-BMP-2, which comprises the following steps:
   (1) a step of producing Ala-BMP-2 by a prokaryote;
   (2) a step of dissolving Ala-BMP-2 obtained in (1) in a strong acid solution; and
   (3) a step of freeze-drying the strong acid solution obtained in (2);
[5] the method of [4], wherein the strong acid is at least one kind selected from the group consisting of hydrochloric acid, citric acid, trifluoroacetic acid and mesyl acid;
[6] a method of improving the solubility of Ala-BMP-2, which comprises the following steps:
   (1') a step of dissolving Ala-BMP-2 in a strong acid solution; and
   (2') a step of freeze-drying the strong acid solution obtained in (1');
[7] the method of [6], wherein the strong acid is at least one kind selected from the group consisting of hydrochloric acid, citric acid, trifluoroacetic acid and mesyl acid;
[8] a kit for regenerating or treating bone, comprising a freeze-dried preparation obtained by freeze-drying a solution containing Ala-BMP-2 and a strong acid, and a calcium phosphate bone prosthetic material in different containers.

### Effect of the Invention

The present invention simultaneously solves the problems of coagulation, solubility and preservation stability of Ala-BMP-2. As a result, Ala-BMP-2 can be used for regenerating or treating bone.

Conventionally, BMP-2 is known to easily dissolve at around pH4, and a freeze-dried preparation of BMP-2 dialyzed against pure water easily dissolves in an acidic solution. However, such freeze-dried preparation does not easily dissolve in pure water, and does not dissolve in saline. In consideration of the use in actual clinical practice, therefore, a BMP-2 preparation superior in the dissolution property in these solvents has been essential.
The freeze-dried preparation of the present invention shows extremely good dissolution property (redissolution property) in pure water, and therefore, quickly dissolves in water for injection and the like to provide a BMP-2 preparation even when in-use formulation of BMP-2 is required during surgery. In addition, the obtained redissolved solution shows low viscosity and is also superior in workability.

Furthermore, since the freeze-dried preparation of the present invention can be easily dissolved in solvents (water for injection etc.) generally used in clinical practice, a particular solvent safer for the body does not need to be prepared, which is convenient and superior from economical aspects.

### Description of Embodiments

The present invention is explained in the following.

### (I) bone morphogenetic protein-2

"BMP-2" belongs to the BMP family or the GDF family, has a binding activity to type I and type II receptors of BMP-2, and has an ability to induce bone morphogenesis, or an ability to regenerate and repair bone (hereinafter these are collectively indicated as "BMP-2 activity").
While the animal species of BMP-2 in the present invention is not particularly limited, it is preferably human.

The "Ala-BMP-2" of the present invention is an active type human BMP-2 homodimer added with an alanine residue to the N-terminus.
As stated in the above-mentioned Background Art section, Ala-BMP-2, like BMP-2, has highly conserved cysteine residues that form 3 disulfide bonds called cysteine knots, and one intermolecular disulfide bond. Ala-BMP-2 is constituted with one cysteine knot, one α-helix and at least 4 β-sheets, per 1 molecule, whereby the monomer can form a dimer. Similar to BMP-2, Ala-BMP-2 forms a homodimer and becomes an active type.

As mentioned above, BMP-2 is synthesized as precursor BMP-2 of an inactive type homodimer that underwent sugar chain modification in a mammalian cell, extracellularly secreted and processed by a protease to be a mature protein of an active type homodimer.
The nucleotide sequence and amino acid sequence of human BMP-2 are registered in GenBank (NCBI, US), and has an Accession Number of NM_001200.

Particularly, Ala-BMP-2 of the present invention is a protein added with an alanine residue to the N-terminus of the amino acid sequence of human BMP-2 homodimer, and free of a sugar chain. The sequence of each monomer (mature type human BMP-2) corresponds to the 114 residues from the 2nd residue to the 115 residue of SEQ ID NO: 1.

While the production method of BMP-2 in the present invention is not particularly limited as long as BMP-2 has the above-mentioned structure, for example, Ala-BMP-2 can be produced by expressing a gene (SEQ ID NO: 2) encoding Met-Ala-BMP-2 added with methionine residue at the N-terminus in a prokaryote. The inventors have confirmed that the recombinant Ala-BMP-2 produced by this method has Ala for all N-terminal amino acid sequences.

BMP-2 in the present invention can be produced by a gene recombination technique using a vector. For example, when Ala-BMP-2 is produced, the vector can contain, for example, a regulatory sequence, a selection marker sequence and the like, in addition to a DNA encoding Met-Ala-BMP-2 or a DNA encoding Ala-BMP-2, which is added with a secretion signal sequence.
The regulatory sequence includes, for example, promoter, enhancer, terminator, replication origin, Shine-Dalgarno (SD) sequence and the like. Examples of the promoter include T3 promoter, T5 promoter, T7 promoter, SP6 promoter, PL promoter, PR promoter and the like derived from phage, and 1ac promoter, trp promoter, alkaline protease promoter, α-amylase promoter and the like derived from bacterium. In addition, the selection marker includes, for example, drug resistance gene (ampicillin resistance gene, neomycin resistance gene, kanamycin resistance gene etc.) and the like.

Examples of the above-mentioned vector include plasmid, phage and the like, more specifically pBR type, pUC type, pBluescript, bacteriophage and the like.
The host cell for producing BMP-2 of the present invention is preferably a prokaryotic cell, which includes, for example, bacteria such as *Escherichia coli, Bacillus subtilis, Pseudomonas* bacterium and the like, preferably *Escherichia coli.*
Prokaryotic cell can be cultured under known culture conditions for *Escherichia coli, Bacillus subtilis, Pseudomonas* bacterium and the like. Culture is performed in a liquid medium with an optimal pH, which contains a carbon source, a nitrogen source, a trace element and the like, at a culture temperature suitable for the cell for a culture time suitable for the cell. The carbon source includes, for example, starch, bran, glucose, lactose, sucrose and the like. The nitrogen source includes, for example, yeast extract, peptone, fish meat extract, urea, ammonium salt, amino acid and the like. The inorganic salt and trace element includes, for example, metal ions such as iron ion, copper ion, zinc ion, magnesium ion, potassium ion, sodium ion, manganese ion, calcium ion and the like, and the like. The culture temperature is generally within the range of 20 - 40°C.

BMP-2 in the present invention can be recovered from a solution resulting from rupture of a transformed host cell and extraction thereof. When a recombinant protein is genetically engineered to be capable of being secreted to the outside of a cell, the object protein can also be recovered from the medium. Then, the recovered protein is refolded and applied to a purification treatment. Purification can be performed by affinity chromatography such as heparin affinity chromatography and the like, gel filtration chromatography, ion exchange chromatography, HPLC, salting out, dialysis, ultrafiltration, electrophoresis, isoelectric focusing and the like, whereby BMP-2 can be obtained. One example of the recovery of Ala-BMP-2 is Ala-BMP-2 recovery method described in non-patent document 1.

### (II) BMP-2 freeze-dried preparation

The freeze-dried preparation of the present invention is a BMP-2 freeze-dried preparation having superior stability, which is obtained by freeze-drying a solution containing a protein added with an alanine residue to the N-terminus of active type BMP-2 and a strong acid.

Particularly, preferred as the freeze-dried preparation of the present invention is a BMP-2 freeze-dried preparation having superior stability and containing Ala-BMP-2 stabilized with a strong acid as an active ingredient, which is obtained by freeze-drying a solution containing Ala-BMP-2 and a strong acid. The "Ala-BMP-2 freeze-dried preparation" which is a preferable example of the present invention is described below.

For production of the Ala-BMP-2 freeze-dried preparation of the present invention, firstly, Ala-BMP-2 is produced by a prokaryote and recovered as mentioned above. The "(1) a step of producing Ala-BMP-2 by a prokaryote, and a method of recovery of Ala-BMP-2" are as mentioned above.

Secondly, the recovered Ala-BMP-2 is directly dissolved in a strong acid solution, recovered Ala-BMP-2 is subjected to ultrafiltration with or dialysis against a strong acid solution, or recovered Ala-BMP-2 is subjected to gel filtration operation and the like to give a strong acid solution of Ala-BMP-2. The "(2) step of dissolving Ala-BMP-2 obtained in Ala-BMP-2 production step (1) in a strong acid solution" is described in detail below.

Examples of the strong acid solution include aqueous strong acid solutions such as aqueous hydrochloric acid solution, aqueous trifluoroacetic acid (TFA) solution, aqueous mesyl acid solution, aqueous phosphoric acid solution, aqueous sulfuric acid solution, aqueous citric acid solution and the like. Preferred is an aqueous strong acid solution having high volatilizability and low hygroscopicity of the strong acid salt, more preferred are an aqueous hydrochloric acid solution, an aqueous citric acid solution, an aqueous trifluoroacetic acid solution and an aqueous mesyl acid solution, and most preferred is an aqueous hydrochloric acid solution.

While the pH of a strong acid solution somewhat varies depending on the measurement environment (for example, apparatus, water, temperature and the like), it is not less than about 0.2 and not more than about 5.1, preferably not less than about 0.2 and not more than about 4.6, more preferably not less than about 3.7 and not more than about 4.6, when measured at 25°C. When pH is lower than 0.2 (i.e., acidic range side) and a freeze-dried form obtained by a freeze-dry operation to follow is redissolved, pH becomes too low to prevent convenient handling thereof. In addition, the solubility of Ala-BMP-2 may decrease, and Ala-BMP-2 itself may be degraded. On the other hand, when pH is higher than 5.1 (i.e., neutral range side), Ala-BMP-2 may not be dissolved.
For example, the concentration of hydrochloric acid when an aqueous hydrochloric acid solution in such pH range is to be adjusted can be selected from the concentration range of 0.01 mM - 330 mM. In addition, such aqueous hydrochloric acid solution in such concentration range can be produced by diluting pharmaceutically acceptable hydrochloric acid with sterile water.

The concentration of Ala-BMP-2 in a strong acid solution is not particularly limited as long as it does not produce precipitation and permits dissolution. It is generally 1 mg/ml - 20 mg/ml, preferably 1 mg/ml - 15 mg/ml, more preferably 1 mg/ml - 5 mg/ml.

An Ala-BMP-2 strong acid solution is prepared by adding small portions of Ala-BMP-2 to a stirring strong acid solution (100-fold volume) adjusted to the above-mentioned concentration or pH range. In this case, the temperature of the strong acid solution is about 0°C - about 30°C, preferably about 4°C - about 20°C, more preferably about 4°C - about 10°C.
While an Ala-BMP-2 strong acid solution may be prepared by simply adding Ala-BMP-2 to a strong acid solution, it is preferably prepared by further subjecting the solution to an ultrafiltration step or a dialysis step. As a method of ultrafiltration or dialysis, one known per se can be applied. For example, after an ultrafiltration step including repeating dilution and concentration of an Ala-BMP-2 strong acid solution about 3 times, Ala-BMP-2 can be dissolved in a strong acid solution. As methods of dilution and concentration, those known per se can be applied.
In addition, an Ala-BMP-2 strong acid solution can also be prepared by, for example, subjecting 100-fold volume of a strong acid solution prepared to fall within the above-mentioned concentration or pH range to dialysis about 3 times. The condition during the dialysis can be appropriately set by those of ordinary skill in the art.

Subsequent to the above-mentioned dissolution step, the obtained Ala-BMP-2 strong acid solution is subjected to a freeze-dry step to remove water therefrom. (3): a step of freeze-drying the strong acid solution obtained in (2) is described in detail below.
The freeze-dry step includes a step of evaporating water under vacuum after freezing an Ala-BMP-2 strong acid solution. As a method of freeze-drying, a conventionally-known method can be employed, or a known freeze dryer may be used. Alternatively, each device such as a cooling trap, a manifold, a cock, a desiccator bottle, a pump and the like may be combined and used as a freeze dryer. These devices are commercially available from, for example, TAITEC.
By freeze-drying an Ala-BMP-2 strong acid solution in this manner, a freeze-dried form is obtained. This freeze-dried form is hereinafter indicated as an "Ala-BMP-2 freeze-dried preparation" and the like.

The Ala-BMP-2 freeze-dried preparation of the present invention is superior in stability, and does not substantially lose BMP-2 activity even after freeze-drying. Therefore, the freeze-dried preparation of the present invention does not require addition of excipient, stabilizer and the like generally contained in preparations and the like, and may be a freeze-dried form of Ala-BMP-2 per se. However, addition of other components is not limited as long as the effect of the present invention is not impaired.
For example, when excipient is added as other component, it can be appropriately selected from conventionally-known excipients, and examples thereof include sugars such as sucrose, trehalose, raffinose, mannitol, dextran and the like, and amino acids such as arginine, histidine, glycine, serine, proline and the like. Furthermore, surfactants and functional substances (e.g., protein, nucleic acid and the like) may also be added.

In the present invention, Ala-BMP-2 is characteristically produced by a prokaryotic cell (preferably *Escherichia coli*), and therefore, is a protein free of a sugar chain. An Ala-BMP-2 freeze-dried preparation produced by the method of the present invention can be stably preserved for not less than 3 months at not more than 45°C, preferably 4 - 25°C, more preferably a low temperature of 4°C or 5°C. Particularly, the Ala-BMP-2 freeze-dried preparation of the present invention can be stably preserved even when chill- (5°C) or freeze-preserved in the form of a freeze-dried solid for 3 months, and is not substantially deactivated.

Here, "stably preserved" means a state substantially free of degradation of protein by an electrophoresis measurement (see the below-mentioned Examples), and/or preservation while substantially maintaining a binding ability to an activine type IIB receptor (ActRIIB), which is a BMP receptor, by a Biacore measurement (GE Healthcare; see the below-mentioned Examples). The stability can also be confirmed by reversed-phase HPLC (e.g., C4 column; mobile phase A,0.1% TFA; mobile phase B, 100% acetonitrile; flow rate, 1 ml/min; gradient, 0% A to 100% B, 20 min).
Preferably, "stably preserved" means preservation while substantially further maintaining BMP activity such as bone induction activity and the like.

The Ala-BMP-2 freeze-dried preparation of the present invention can not only be preserved stably under the above-mentioned conditions but also is not substantially deactivated even when freeze-thawed, retains complete BMP activity (e.g., bone induction activity) and does not substantially lose BMP activity even when preserved at 40°C.
As mentioned above, an Ala-BMP-2 freeze-dried preparation produced by the method of the present invention has improved preservation stability.

Moreover, the Ala-BMP-2 freeze-dried preparation of the present invention is characterized in that the pH range, when it is redissolved in water to be restored to the volume before freeze-drying, is generally not less than 3 and not more than 5, preferably not less than 3.7 and not more than 4.6. When an aqueous Ala-BMP-2 solution with such pH range is obtained and the below-mentioned bone prosthetic material is impregnated with the solution, Ala-BMP-2 adsorbs to calcium phosphate forming the bone filler. When such bone filler is embedded in the damaged bone area, a bone prosthetic material having bone regeneration capability is easily obtained. Therefore, the Ala-BMP-2 freeze-dried preparation of the present invention is extremely useful.

Conventional Ala-BMP-2 freeze-dried preparations show low solubility in water and saline (easily coagulated), and lack stability as BMP (see non-patent document 1). Therefore, conventional Ala-BMP-2 freeze-dried preparations require addition of various additives to enhance solubility and stability thereof.
However, the Ala-BMP-2 freeze-dried preparation of the present invention characteristically maintains solubility and stability even without various additives, for example, stabilizers such as saccharide, protein, surfactant, amino acid and the like, buffering agents such as aqueous glycine-buffered solution and the like. As such, the Ala-BMP-2 freeze-dried preparation produced by the method of the present invention is extremely useful as compared to conventional Ala-BMP-2 freeze-dried preparations, since it substantially prevents easy coagulation and shows high preservation stability.

In the production method of the Ala-BMP-2 freeze-dried preparation of the present invention, the step (2) for dissolving in a strong acid solution and the step (3) for freeze-drying the strong acid solution after dissolution are essential steps to be included in a method for improving solubility of Ala-BMP-2.
Accordingly, the present invention also provides a method of improving solubility of Ala-BMP-2, which comprises the following steps:
(1') a step of dissolving Ala-BMP-2 in a strong acid solution; and
(2') a step of freeze-drying the strong acid solution obtained in (1').

In the production method of the Ala-BMP-2 freeze-dried preparation of the present invention, moreover, the step (2) for dissolving in a strong acid solution and the step (3) for freeze-drying the strong acid solution after dissolution are essential steps to be included in a method for improving preservation stability of Ala-BMP-2.
Accordingly, the present invention also provides a method of improving preservation stability of Ala-BMP-2, which comprises the following steps:
(1") a step of dissolving Ala-BMP-2 in a strong acid solution; and
(2") a step of freeze-drying the strong acid solution obtained in (1").

The Ala-BMP-2 freeze-dried preparation of the present invention can be redissolved in a solution for redissolution added when in use. As the solution for redissolution, water (e.g., water for injection, sterile water, bacteriostasis water), aqueous solution (e.g., saline), buffered solution (e.g., aqueous acetic acid-buffered solution, aqueous phthalic acid-buffered solution, aqueous glycine-buffered solution) and the like can be used. The freeze-dried preparation of the present invention has superior solubility in these solutions for redissolution, and an aqueous Ala-BMP-2 solution satisfying the need can be prepared in a short time. Such aqueous Ala-BMP-2 solution also maintains sufficient BMP-2 activity, and can exhibit a superior bone regeneration and treatment effect.
When the below-mentioned bone filler is impregnated with the aqueous Ala-BMP-2 solution, Ala-BMP-2 adsorbs to calcium phosphate component forming the bone filler. When such bone filler is embedded in the damaged bone area, a bone prosthetic material having bone regeneration capability is easily obtained. As a result, bone regeneration in or bone treatment of a bone fracture area and a bone defect area becomes possible.

### (III) Pharmaceutical preparation for bone regeneration

The present invention further provides a pharmaceutical preparation containing an Ala-BMP-2 freeze-dried preparation.
Since the pharmaceutical preparation of the present invention contains the Ala-BMP-2 freeze-dried preparation of the present invention, it has higher preservation stability than conventional BMP freeze-dried preparations. Therefore, excipient, carrier, additive and the like used for general pharmaceutical preparations are substantially unnecessary, and the preparation can be directly used for a medical use. However, it is also possible to add, where necessary, a substance such as pharmaceutically acceptable, excipient, carrier, additive, other medicament having bone induction activity, therapeutic agent for bone disease and the like to the pharmaceutical preparation of the present invention.

Since the pharmaceutical preparation of the present invention has unimpaired BMP-2 activity, particularly bone induction activity that BMP-2 has, it can be used for regeneration, repair or treatment of bone. For bone regeneration, bone morphogenetic protein (BMP), stem cell and scaffold for bone regeneration (bone prosthetic material) are necessary.
Examples of the scaffold for bone regeneration (bone prosthetic material) include synthetic polymers such as polylactic acid (PLA), polylactic glycolate (PLGA), PLA-PEG, PLGA-PEG (PEG is polyethylene glycol) and the like, natural polymers such as hyaluronic acid, collagen, atelocollagen and the like, calcium phosphates such as hydroxyapatite, β-tricalcium phosphate (β-TCP), α-TCP and the like, and the like.
In fact, a porous scaffold (having a form of, for example, granule, spongy, block, gel and the like) and the pharmaceutical preparation of the present invention (or the Ala-BMP-2 freeze-dried preparation of the present invention) are mixed with an aqueous solution, and the mixture is filled in an affected part of bone, for example, bone fracture area, defect area, damaged area and the like. As a result, Ala-BMP that has adsorbed to the scaffold acts on endogenous stem cells, and the bone tissue is regenerated by the induction of differentiation of the stem cell. When the scaffold is a granule, one having a particle size permitting passage through 3 - 5 mm mesh arrow is generally used for orthopedic surgery, and one having a particle size permitting passage through 0.5 - 1.5 mm mesh arrow is generally used for dentistry. The pharmaceutical preparation of the present invention includes an embodiment of a mixture of Ala-BMP-2 and scaffold, an embodiment of Ala-BMP-2 applied to scaffold, an embodiment of Ala-BMP-2 adsorbed to scaffold and the like.

An optimal single clinical dose (single placement or injection) of Ala-BMP-2 freeze-dried preparation to human in the pharmaceutical preparation of the present invention varies depending on the administration site, size of defect, scaffold to be combined (collagen, bone prosthetic material, polymer gel etc.). It is generally about 0.2 - 3 mg (weight of Ala-BMP-2) per 1cm³ of bone filling volume. For example, when the bone filling volume has a width range of about 0.5 - 20 cm³, it is within the range of 0.1 - 60 mg/patient. However, such single clinical dose may change depending on various conditions of patients such as sex, age, body weight, severity and the like.

The pharmaceutical preparation of the present invention can be used for regeneration and filling of bone defect, early healing of bone fracture, regeneration and treatment of damaged areas of bone such as cartilage, tendon, ligament, intervertebral disk, meniscus, alveolar bone and the like, and the like. Since Ala-BMP-2, which is the active ingredient of the pharmaceutical preparation of the present invention, shows high bone induction ability, is less-invasive, and affords advantages of stable quality control and stable supply, its use for the above-mentioned medical applications is expected.

### (IV) Kit for bone regeneration or bone treatment

The present invention provides a kit for regeneration or treatment of bone, comprising an Ala-BMP-2 freeze-dried preparation, and a calcium phosphate bone prosthetic material in different containers.
Examples of the calcium phosphate bone prosthetic material include, but are not limited to, bone prosthetic materials containing calcium phosphate as a main component such as hydroxyapatite, β-TCP and α-TCP and the like.
The kit of the present invention may further contain an instruction manual, a syringe containing a solution for redissolution such as water for injection and the like, a sterilized tray, a sterilized spatula and the like. In this case, Ala-BMP-2 is dissolved in water (aqueous solution) in a syringe, which is evenly sprayed on a calcium phosphate bone prosthetic material (granule) in a tray, and they are mixed with a spatula. Such kit intends reconstitution or redissolution of Ala-BMP-2 immediately before bone (regeneration) treatment. Since the Ala-BMP-2 freeze-dried preparation of the present invention dissolves in water (aqueous solution) in a short time, and the obtained solution has low viscosity, it is superior in that it can improve work efficiency in clinical practice.

### Examples

The present invention is explained in more detail by referring to the following Examples, Experimental Examples and the like, which do not limit the scope of the present invention.

### Example 1: Preparation of Ala-BMP-2

Ala-BMP-2 was prepared according to the method described in non-patent document 1. That is, *Escherichia coli* BL-21 was utilized to express a DNA encoding Met-Ala-BMP-2 (SEQ ID NO: 1), and each operation of the recovery of inclusion bodies, refolding of protein, and recovery of dimer was performed by a combination of known methods. Ala-BMP-2 dissolved in 4M urea solution was placed in a dialysis tube with a cut-off molecular weight of 3500, and dialysis was performed using various aqueous acid solutions described in the following Experimental Example 1 (Preparation Examples 1 - 2, Comparative Example 1, control materials 1 and 2). The dialysis solution was recovered, the concentration was confirmed at OD 278 nm and concentrated by ultrafiltration. Amicon (registered trade mark) Ultra-15 centrifugal filter unit 10K (manufactured by Millipore) was used for the concentration step.

### Example 2: Freeze-dry

The sample obtained in the concentration step of Example 1 was recovered, the concentration was confirmed at OD 278 nm, dispensed at 5 mg Ala-BMP-2/1.5 ml tube, and frozen at -80°C overnight or longer. The frozen sample was freeze-dried with a freeze dryer.

### Experimental Example 1: Redissolution test

Dialysis was performed in the dialysis step shown in Example 1, using various aqueous acid solutions (dialysis solutions) shown in Table 1 below.
The state of each sample during dialysis, easiness of freeze-drying, and resolubility of the obtained freeze-dried preparation in water were observed. The freeze-dried preparation was dissolved in water for injection at a concentration of 5 mg/ml and used for the redissolution.

**Table 1**

| | dialysis conditions | state during dialysis | freeze-dry | resolubility of freeze-dried preparation |
|---|---|---|---|---|
| Prepar. Ex. 1 | 0.5 mM hydrochloric acid, 3 times | Precipitated immediately after start of dialysis but quickly redissolved within 30 min | easy | Infiltrated immediately after addition of water for injection, and mostly redissolved by standing for about 5 min. More easily redissolved using stirring operation by vortex etc. |
| Prepar. Ex. 2 | 0.8 mM citric acid, 3 times | precipitated immediately after start of dialysis but redissolved in about 3 hr | easy | Infiltrated immediately after addition of water for injection, and mostly redissolved by standing for about 5 min. More easily redissolved using stirring operation by vortex etc. |
| Compar. Ex. 1 | 0.06% acetic acid, 3 times | precipitated immediately after start of dialysis but quickly redissolved within 30 min | easy | Infiltrated by addition of water for injection, but about half cannot be redissolved by standing only for about 20 min. Redissolved using stirring operation by vortex etc., but viscosity is high and operation becomes complicated. |
| Control sample X | 3 times with distilled water (method described in non-patent document 1) | precipitated immediately after start of dialysis, and did not redissolve | - | - |
| | once with 0.001% trifluoroacetic acid→3 times with distilled water (insoluble material of *1 as starting sample) | quickly redissolved within 30 min | easy | Infiltrated immediately after addition of water for injection, and mostly redissolved by standing for about 20 min. More easily redissolved using stirring operation by vortex etc. |
| Control sample Y | 3 times with distilled water (method described in non-patent document 1) | precipitated immediately after start of dialysis, and did not redissolve | - | - |
| | once with 0.5 mM hydrochloric acid→ 3 times with distilled water (insoluble material of *1 as starting sample) | quickly redissolved within 30 min | easy | Infiltrated by addition of water for injection, but about half cannot be redissolved by standing only for about 20 min. Redissolution is possible by stirring operation by vortex etc., though it takes time. |

As shown in Table 1, Preparation Example 1 and Preparation Example 2 using an aqueous strong acid solution for dialysis showed redissolution, though precipitation occurred after the start of dialysis, and freeze-dry was easy. Preparation Example 1 and Preparation Example 2 using a freeze-dried preparation showed good resolubility.
On the other hand, Comparative Example 1 using an aqueous weak acidic solution (0.06% acetic acid) for dialysis showed a similar behavior as in Preparation Example 1 during dialysis and freeze-dry step, but evaluation of resolubility of the freeze-dried preparation revealed high viscosity, and redissolution was not possible without stirring.
In control sample X and control sample Y for which dialysis was performed according to a conventional method described in non-patent document 1, precipitation occurred during dialysis and the precipitate was not redissolved. However, these were dialyzed against an aqueous strong acid solution and dialyzed against distilled water, whereby a freeze-dried preparation with high resolubility could be obtained.
Furthermore, Preparation Example 1 and Preparation Example 2 showed a clearly faster dissolution rate than control sample X and control sample Y.

### Experimental Example 2: storage stability evaluation

### (1) Preparation of Ala-BMP-2

Using the method described in Example 1, Ala-BMP-2 was produced and recovered. Then, substitution with 0.5 mM/L hydrochloric acid was performed by ultrafiltration/dialysis filtration, and Ala-BMP-2 concentration was adjusted to 2.5 mg/ml.

### (2) freeze-dry

The sample with an adjusted concentration in the above-mentioned (1) was recovered, 10 mg was dispensed into a vial (diameter 24.5 mm×53 mm) and frozen at -50°C overnight or longer. The frozen sample was freeze-dried by a freeze dryer. The freeze dryer was the same as that used in the above-mentioned Example 2.

### (3) Storage stability test

The evaluation items and evaluation method relating to the storage stability are as shown in Table 2.

**Table 2**

| | |
|---|---|
| property | According to the Japanese Pharmacopoeia/general principle. |
| time for dissolution | Water for injection was added, "gentle stirring for 5 seconds, and visual observation for 25 seconds" was repeated, and the dissolution time was measured. |
| postdissolution property | According to the Japanese Pharmacopoeia/general principle. |
| pH | According to the Japanese Pharmacopoeia/2.54 pH measurement method. |
| osmotic pressure | According to the Japanese Pharmacopoeia/2.47 osmotic pressure measurement method (osmolar concentration measurement method). |
| gel filtration chromatography | According to the Japanese Pharmacopoeia/2.01 liquid chromatography. |
| | (column used: Tosoh Corporation, TSKgel SuperSW2000) |
| reducing SDS-PAGE | According to the Japanese Pharmacopoeia/reference information 6. SDS polyacrylamide electrophoresis. |
| | Utilized for confirmation of molecular weight. (densitometer used: Personal Densitometer SI manufactured by GE Healthcare Japan Corporation) |
| non-reducing SDS-PAGE | According to the Japanese Pharmacopoeia/reference information 6. SDS polyacrylamide electrophoresis. |
| | Utilized for confirmation of purity. (densitometer used: Personal Densitometer SI manufactured by GE Healthcare Japan Corporation) |
| protein quantification (UV method) | According to the Japanese Pharmacopoeia/2.24 ultraviolet visible absorbance measurement method. |
| C2C12 cell bioassay* | Cell bioassay using C2C12 cell line (mouse myoblast). BMP-2 was added to C2C12 cell known to differentiate into myotube cell and the cell was cultivated. The differentiation into myotube cell was suppressed and differentiation into osteoblast occurs. Therefore, when differentiation into osteoblast is observed, BMP-2 is judged to have activity. |
| Biacore assay* | Ligand - receptor binding reaction test using BIAcore 2000. |
| | Evaluation of binding ability of activine type IIB receptor (ActRIIB) which is a BMP receptor enables confirmation of presence or absence of activity of BMP-2. |
| | (analyte: Ala-BMP-2, ligand: ActRIIBecd) |
| insoluble foreign substance | According to the Japanese Pharmacopoeia/6.06 insoluble foreign substance test for injection. |

| | |
|---|---|
| * relative value to provisional reference substance of Ala-BMP-2 active pharmaceutical ingredient (manufactured by Toyobo Biologics Inc.) | |

The evaluation results are shown in Table 3.

**Table 3**

| test item | at start of test | preservation at 5±3°C | preservation at 25+2°C, relative humidity 60±5% | preservation at 40±2°C, relative humidity 75±5% |
|---|---|---|---|---|
| | | preservation for 3 months | preservation for 3 months | preservation for 3 months |
| property | white mass | white mass | white mass | white mass |
| dissolution time | 2 min 00 sec | 2 min 30 sec | 2 min 30 sec | 2 min 30 sec |
| property after dissolution | colorless clear solution | colorless clear solution | colorless clear solution | colorless clear solution |
| pH | 4.1 | 3.9 | 3.9 | 4.0 |
| osmotic pressure | 3 mOsm | 4 mOsm | 4 mOsm | 4 mOsm |
| gel filtration chromatography* | 100% | 99.9% | 99.8% | 99.5% |
| reductive SDS-PAGE* | 98.2% | 98.2% | 97.5% | 95.6% |
| non-reductive SDS-PAGE* | 98.2%, | 98.2% | 97.5% | 96.6% |
| protein quantification (UV method) | 2.5 mg/mL | 2.5 mg/mL | 2.4 mg/mL | 2.4 mg/mL |
| C2C12 cell bioassay* | 101.6% | 101.0% | 90.2% | 103.8% |
| Biacore assay* | 98.2% | 100.3% | 98.9% | 97.7% |
| insoluble foreign substance | undetectable | undetectable | undetectable | undetectable |

| | | | | |
|---|---|---|---|---|
| * relative value to provisional reference substance of Ala-BMP-2 active pharmaceutical ingredient (manufactured by Toyobo Biologics Inc.) as 100%. | | | | |

As shown in Table 3, the properties and dissolution property of freeze-dried preparation were not impaired under any preservation conditions, and Ala-BMP-2 activity after redissolution was also maintained sufficiently. Moreover, the property and Ala-BMP-2 activity were not impaired even under relatively harsh preservation conditions of 40±2°C, relative humidity 75±5%. That is, the freeze-dried preparation of the present invention was shown to have a superior preservation stability.

### Example 3: Production of Ala-BMP-2 freeze-dried preparation

A solution having the following composition was filled in a 10 mL vial, uniformly mixed, and frozen at -80°C overnight or longer. The frozen sample was freeze-dried by a freeze dryer similar to that in Example 1. The vial was capped with a rubber cap, and the cap was screwed to give a vial-filled freeze-dried preparation.

| | |
|---|---|
| (solution 1) | |
| 0.1 mM hydrochloric acid containing Ala-BMP-2 (5 mg) | 1.0 mL |
| (solution 2) | |
| 0.5 mM hydrochloric acid containing Ala-BMP-2 (5 mg) | 1.0 mL |
| (solution 3) | |
| 0.1 mM citric acid containing Ala-BMP-2 (5 mg) | 1.0 mL |
| (solution 4) | |
| 0.1 mM TFA containing Ala-BMP-2 (5 mg) | 1.0 mL |
| (solution 5) | |
| 0.1 mM mesylic acid containing Ala-BMP-2 (5 mg) | 1.0 mL |

### Industrial Applicability

The present invention has enabled use of Ala-BMP-2 produced by a prokaryotic cell such as *Escherichia coli* and the like for clinical purposes. Since Ala-BMP-2 is a protein useful for regeneration and treatment of bone, its role in the clinical field is expected.

### [Sequence Listing Free Text]

SEQ ID NO: 1 is the amino acid sequence of human bone morphogenetic protein-2 added with an alanine residue to the N-terminus.
SEQ ID NO: 2 is the sequence of a DNA encoding human bone morphogenetic protein-2 added with an alanine residue to the N-terminus.

## Claims

1. A freeze-dried preparation obtained by freeze-drying a solution containing Ala-BMP-2 and a strong acid.

2. The freeze-dried preparation according to claim 1, wherein the strong acid is at least one kind selected from the group consisting of hydrochloric acid, citric acid, trifluoroacetic acid and mesyl acid.

3. The freeze-dried preparation according to claim 1 or 2, wherein the strong acid has pH 0.2 - 5.1 at 25°C.

4. A method of producing a freeze-dried preparation containing Ala-BMP-2, which comprises the following steps:
(1) a step of producing Ala-BMP-2 by a prokaryote;
(2) a step of dissolving Ala-BMP-2 obtained in (1) in a strong acid solution; and
(3) a step of freeze-drying the strong acid solution obtained in (2).

5. The method according to claim 4, wherein the strong acid is at least one kind selected from the group consisting of hydrochloric acid, citric acid, trifluoroacetic acid and mesyl acid.

6. A method of improving the solubility of Ala-BMP-2, which comprises the following steps:
(1') a step of dissolving Ala-BMP-2 in a strong acid solution; and
(2') a step of freeze-drying the strong acid solution obtained in (1').

7. The method according to claim 6, wherein the strong acid is at least one kind selected from the group consisting of hydrochloric acid, citric acid, trifluoroacetic acid and mesyl acid.

8. A kit for regenerating or treating bone, comprising a freeze-dried preparation obtained by freeze-drying a solution containing Ala-BMP-2 and a strong acid, and a calcium phosphate bone prosthetic material in different containers.


SEQUENCE LISTING

<110>  OSTHEOPHARMA INC.

<120>  Lyophilized formulation of recombinant human Bone Morphogenetic
       Protein-2

<130>  091605

<160>  2

<170>  PatentIn version 3.3

<210>  1
<211>  115
<212>  PRT
<213>  Artificial

<220>
<223>  Mature human Bone Morphogenetic Protein-2 wherein N-terminal is
       modified with alanine residue

<400>  1

Ala Gln Ala Lys His Lys Gln Arg Lys Arg Leu Lys Ser Ser Cys Lys
1               5                   10                  15

Arg His Pro Leu Tyr Val Asp Phe Ser Asp Val Gly Trp Asn Asp Trp
            20                  25                  30

Ile Val Ala Pro Pro Gly Tyr His Ala Phe Tyr Cys His Gly Glu Cys
        35                  40                  45

Pro Phe Pro Leu Ala Asp His Leu Asn Ser Thr Asn His Ala Ile Val
    50                  55                  60

Gln Thr Leu Val Asn Ser Val Asn Ser Lys Ile Pro Lys Ala Cys Cys
65                  70                  75                  80

Val Pro Thr Glu Leu Ser Ala Ile Ser Met Leu Tyr Leu Asp Glu Asn
                85                  90                  95

Glu Lys Val Val Leu Lys Asn Tyr Gln Asp Met Val Val Glu Gly Cys
            100                 105                 110

Gly Cys Arg
        115

<210>  2
<211>  348
<212>  DNA
<213>  Artificial

<220>
<223>  DNA cording Mature human Bone Morphogenetic Protein-2 wherein

N-terminal is modified with alanine residue

<400>  2
atggctcaag ccaaacacaa acagcggaaa cgccttaagt ccagctgtaa gagacaccct      60

ttgtacgtgg acttcagtga cgtggggtgg aatgactgga ttgtggctcc cccggggtat     120

cacgcctttt actgccacgg agaatgccct tttcctctgg ctgatcatct gaactccact     180

aatcatgcca ttgttcagac gttggtcaac tctgttaact ctaagattcc taaggcatgc     240

tgtgtcccga cagaactcag tgctatctcg atgctgtacc ttgacgagaa tgaaaaggtt     300

gtattaaaga actatcagga catggttgtg gagggttgtg ggtgtcgc                  348

SEQUENCE LISTING

<110>  OSTHEOPHARMA INC.

<120>  Lyophilized formulation of recombinant human Bone Morphogenetic
       Protein-2

<130>  091605

<140>  PCT/JP10/64967
<141>  2010-09-01

<160>  2

<170>  PatentIn version 3.3

<210>  1
<211>  115
<212>  PRT
<213>  Artificial

<220>
<223>  Mature human Bone Morphogenetic Protein-2 wherein N-terminal is
       modified with alanine residue

<400>  1

Ala Gln Ala Lys His Lys Gln Arg Lys Arg Leu Lys Ser Ser Cys Lys
1               5                   10                  15

Arg His Pro Leu Tyr Val Asp Phe Ser Asp Val Gly Trp Asn Asp Trp
            20                  25                  30

Ile Val Ala Pro Pro Gly Tyr His Ala Phe Tyr Cys His Gly Glu Cys
        35                  40                  45

Pro Phe Pro Leu Ala Asp His Leu Asn Ser Thr Asn His Ala Ile Val
    50                  55                  60

Gln Thr Leu Val Asn Ser Val Asn Ser Lys Ile Pro Lys Ala Cys Cys
65                  70                  75                  80

Val Pro Thr Glu Leu Ser Ala Ile Ser Met Leu Tyr Leu Asp Glu Asn
                85                  90                  95

Glu Lys Val Val Leu Lys Asn Tyr Gln Asp Met Val Val Glu Gly Cys
            100                 105                 110

Gly Cys Arg
        115

<210>  2
<211>  348
<212>  DNA
<213>  Artificial

<220>
<223>  DNA cording Mature human Bone Morphogenetic Protein-2 wherein
       N-terminal is modified with alanine residue

<400>  2
atggctcaag ccaaacacaa acagcggaaa cgccttaagt ccagctgtaa gagacaccct      60

ttgtacgtgg acttcagtga cgtggggtgg aatgactgga ttgtggctcc cccggggtat     120

cacgcctttt actgccacgg agaatgccct tttcctctgg ctgatcatct gaactccact     180

aatcatgcca ttgttcagac gttggtcaac tctgttaact ctaagattcc taaggcatgc     240

tgtgtcccga cagaactcag tgctatctcg atgctgtacc ttgacgagaa tgaaaaggtt     300

gtattaaaga actatcagga catggttgtg gagggttgtg ggtgtcgc                  348